# EUROPEAN PATENT SPECIFICATION

(11) **EP 2 670 362 B1**
(45) Date of publication and mention of the grant of the patent: **11.03.2015**
(21) Application number: 12703354.6
(22) Date of filing: 30.01.2012
(51) Int. Cl.: A61F 2/95

(54) **STENT DELIVERY DEVICE**
STENTVERABREICHUNGSVORRICHTUNG
DISPOSITIF D'IMPLANTATION D'ENDOPROTHÈSE

(30) Priority: 01.02.2011 FR 1150788; 01.02.2011 US 201161438477 P
(43) Date of publication of application: 11.12.2013
(73) Proprietor: Stentys, 75002 Paris (FR)
(72) Inventor: ATLANI, David, 95280 Jouy Le Moutier (FR); HOJEIBANE, Hikmat, Princetown, New Jersey 08540 (US); ISSENMANN, Gonzague, 92110 Clichy (FR)
(74) Representative: Jeannet, Olivier
(86) International application number: PCT/IB2012/050403
(87) International publication number: WO 2012/104763

(56) References cited:
- WO-A1-2009/005933
- US-A- 5 456 694
- US-A1- 2002 052 640
- US-A1- 2005 288 763
- US-A1- 2007 088 368

## Description

The present invention relates to a stent delivery system.

To reestablish the caliber of a bodily conduit, it is well known to place, in that bodily conduit, a deployable tubular framework with an openwork structure, commonly called a "stent." Such a stent can adopt a radially contracted state, allowing it to be inserted into the bodily conduit, and a radially expanded state, in which it is found when it is implanted and owing to which it reestablishes the caliber of the bodily conduit. The stent can be made from an elastic material making it self-expanding, or can be made from a material making it necessary to perform, in whole or in part, a forced expansion (often called "stent on balloon").

One well-known system for bringing a self-expanding stent to its implantation site and delivering that stent to the site consists of a catheter inserted into the patient and slid into the latter's bodily conduit. This sliding is done until the distal end of the catheter, which comprises the stent in the aforementioned contracted state, is found at the implantation site. Such a catheter comprises a central support comprising the stent on its distal end, and a sheath capable of being slid relative to said central support so as to free the expansion of the stent.

It will be understood that the terms "distal" and "proximal" used in this description should be considered relative to the operator, "distal" meaning remote from the operator (or on the contrary close to the implantation site) and "proximal" meaning close to the operator (or on the contrary remote from the implantation site).

Another well-known system for bringing a stent on balloon toward its implantation site is a catheter comprising an inflatable balloon on which the stent is placed, this balloon making it possible to perform said forced expansion.

The positioning of the stent in the bodily conduit to be treated must be precise, failing which the recalibration of that conduit is ineffective or partially effective. This positioning must be even more precise inasmuch as the stent is no longer movable once it is expanded and bearing against the wall of the bodily conduit. In the case of a self-expanding stent placed using a known catheter, the precise positioning of the stent depends greatly on the dexterity and experience of the operator, since the removal of the sheath is usually done by applying a relative movement between said support and the sheath, involving exerting a pulling force on the sheath; given that this action is done from outside the patient and the catheter itself is inserted into a guide catheter, it tends to cause an uncontrolled movement of the sheath and the catheters during the deployment phase of the stent. Furthermore, the stent being elastically deformed when it is radially contracted, there is a risk of ejection of the stent relative to the catheter during the release of that stent, leading the latter to "jump" from the catheter after a certain degree of removal of the sheath.

The present invention aims to resolve all of these essential drawbacks, by providing a system for delivering a self-expanding stent or a "stent on balloon" that allows precise positioning of the stent in the bodily conduit to be treated.

Another problem that arises with a stent on balloon comprising a coating constituting a medication is that the forward movement of the catheter through the conduit to be treated causes the risk of damaging said coating by friction against the wall of the conduit. In certain anatomies, the wall of the conduit can also be damaged by the friction of the stent against it.

The present invention also aims to resolve these drawbacks, by providing a system for delivering a stent on balloon that makes it possible to avoid contact between the surface of the stent and the bodily conduit to be treated until the stent is deployed.

Document US 2002/052640 describes a system for delivering a stent including a sheath or biocompatible or bio-absorbable filaments for setting the stent. The sheath has a sufficient strength to keep the stent in the contracted state, but is fragile enough to be broken or expanded by inflating the balloon. Such a system, with a non-sliding sheath, does not correspond to the system according to the invention, in which the sheath can slide and in which the aforementioned problem arises of precisely positioning the stent in the bodily conduit to be treated.

Documents WO 2009/005933, US 2005/0288763, US 2007/0088368 or US 5,456,694 describe various other existing systems, also not making it possible to resolve the aforementioned problems.

According to the invention, the system comprises:
- a stent having a proximal end;
- a support comprising the stent engaged thereon, having a longitudinal axis and being equipped with a stop against which the proximal end of the stent is applied;
- a sheath having a distal end, which can be slid relative to said support and said stop, between a distal overlap position of the stent and a proximal release position for the deployment of the stent, and
- a first inflatable balloon situated between said support and the stent, comprising a distal portion that protrudes past the distal end of the sheath, on the distal side thereof, when the latter is in said distal position, and having, in the inflated state, over all the length thereof, or over a part of the length thereof, on the distal side, a transverse section larger than or equal to that of the sheath,
the distal end of the sheath being, in said distal overlap position of the stent, situated back from said distal end, along said longitudinal axis of the support, in the proximal direction, and being situated opposite said distal portion, such that said distal portion protrudes partially past said distal end of the sheath, on the distal side of said end.

Thus, to deliver the stent, said first balloon is first supplied with inflating fluid, which first inflates said distal portion of the balloon and then creates an inflation wave moving in the proximal direction; this inflation wave causes the balloon to come into contact with the distal end of the sheath, then sliding of the sheath along the wall of the balloon, which makes it possible to remove the sheath; at the beginning of that removal, the distal end of the stent is maintained by said inflated distal portion of the balloon, which prevents any risk of the stent "jumping" relative to said support. During this inflation of the balloon and removal of the sheath, the bearing of the proximal end of the stent against said stop results preponderantly in avoiding any risk of removal of the stent at the same time as the sheath.

The removal of the sheath is therefore done without any pulling force having been exerted on said sheath from outside the patient, which avoids the risk of moving the entire system, and therefore the stent, relative to the implantation site. On the contrary, the inflation of said distal portion of the balloon immediately presses the distal end of the stent against the wall of the bodily conduit as of the beginning of removal of the sheath, and therefore performs a certain axial immobilization of the stent relative to said conduit as of that beginning of removal.

As a result, the system according to the invention makes it possible to deliver the stent in a very precise position.

Preferably, the system includes locking means that can be actuated between a locking position and an unlocking position, these locking means being able, in the locking position, to immobilize the sheath in said distal overlap position and, in the unlocking position, to release the sliding of the sheath.

These means are in the locking position during the insertion and sliding of the system in the bodily conduit toward the implantation site, and are actuated in the unlocking position of said sheath when the stent is located opposite said implantation site.

These locking means can comprise a sheath having a length such that it extends outside the patient when the stent is located opposite the implantation site, and means for immobilizing the sheath relative to said support, capable of being controlled from outside the patient.

According to one preferred embodiment of the invention, however, these locking means comprise a second inflatable balloon, fixed in position relative to said support and situated inside the sheath, the inflation of which corresponds to said locking position and the deflation of which corresponds to said unlocking position; in the inflated state, this second balloon is pressed against the inner surface of the sheath so as to generate friction therewith, immobilizing the latter in the axial direction relative to said support, and, in the deflated state, this second balloon is not pressed against the inner surface of the sheath so as to allow the sliding of the latter.

According to another embodiment of the invention, said locking means also comprise a second inflatable balloon fixed in position relative to said support and the inflation of which corresponds to said locking position while the deflation thereof corresponds to said unlocking position; in this case, this second balloon is situated outside the sheath, on the proximal side of a proximal end of said sheath; the second balloon has, in the inflated state, a diameter larger than that of the sheath, so that this second balloon forms a stop against which the proximal end of the sheath bears so as to block the sliding of the sheath; in the deflated state, this second balloon has a diameter smaller than that of the sheath, so that this second balloon does not form a stop for said proximal end of the sheath and therefore does not hinder the sliding of the sheath.

This second balloon is therefore in the inflated state during the insertion and sliding of the system in the bodily conduit of the patient toward the implantation site, thereby locking the sheath in said distal overlap position, and is deflated just before the deployment operation of the stent, so as to release the removal of the sheath.

Said second balloon can in particular be situated close to said stop, on the proximal side thereof.

The distance separating the distal end of said balloon and the distal end of the sheath when the latter is in the distal position, must be greater than 0.5 times the diameter of the sheath; it is preferably comprised between one and two times the diameter of the sheath.

Said first balloon can have a cylindrical shape, with a diameter at least equal to that of the sheath; it can also have a conical shape, whereof the distal portion, with the larger diameter, has a diameter at least equal to that of the sheath; it can also have one or more longitudinal ribs, in particular four equidistant longitudinal ribs, so as to decrease any friction with the sheath; it can also have, in the deflated or folded state, a distal portion having a transverse section larger than that of its proximal portion, this distal portion in particular being able to have a more or less ovoid or hernial shape; it can also have a helical groove in the inflated state, the purpose of which is to reduce the force radially exerted by the balloon on the sheath as well as the friction existing between said balloon and the stent.

This first balloon can also have a shape other than a circular shape in transverse section, for example oval, so as to decrease any friction with the sheath.

According to one possibility, said support comprises a widened distal end piece (commonly called "tip"), capable of favoring the progression of the system in a bodily conduit, and the sheath comprises a sleeve extending its distal end, which is dimensioned so as to extend to said end piece or tip, this sleeve being made from a material more flexible than that of the sheath, not offering resistance to the inflation of said distal portion of said first balloon, or a low resistance to that inflation.

This sleeve thereby allows the system to have a substantially smooth and continuous outer wall between said end piece and said sheath.

According to one possible embodiment of the invention, said support comprises a widened distal end piece, capable of favoring the progression of the system in a bodily conduit, and said distal portion of the first balloon is shaped so as to surround a proximal portion of that end piece and, in the non-inflated state, to extend close to the distal end of the sheath.

Said first balloon, thus shaped, also allows the system to have a substantially smooth and continuous outer wall between said end piece and said sheath.

Preferably, the system includes a proximal inflation restricting element, making it possible to ensure that the inflation of said first balloon starts at said distal portion of the balloon and progresses in the proximal direction.

This restriction element makes it possible to prevent early proximal inflation from occurring, i.e. proximal inflation occurring at the same time as the distal inflation, which would produce a radial force on the sheath, generating tightening thereof capable of hindering, or even preventing, the removal of the sheath.

According to one possible embodiment of the invention, this restriction element is in the form of a sleeve surrounding said balloon in an adjusted manner, one distal end of said sleeve protruding past the distal end of the sheath and being situated back from the distal end of said first balloon in the proximal direction, and this sleeve being longitudinally tearable under the effect of the inflation of this first balloon, so as not to hinder the inflation.

The inflation of said first balloon is thus done from said distal end of this first balloon and progresses in the proximal direction, the proximal portion of said first balloon not being able to inflate, being maintained by the sleeve; this inflation causes the gradual tearing of the sleeve from said distal end of the sleeve, which allows the inflation to continue, in the proximal direction.

Said sleeve can be made from a material with a low coefficient of friction, in particular PTFE (polytetrafluoroethylene). This material favors the engagement of the sleeve on the stent during the assembly of the device, and the removal of said first balloon relative to the stent once the latter is placed.

Said sleeve can in particular comprise a longitudinal notch arranged from its distal end, constituting a beginning of longitudinal tearing.

Preferably, the system includes an element for preventing or restricting the radial expansion of the sheath, also resulting in preventing early proximal inflation of said first balloon from occurring.

According to one possible embodiment of the invention, this expansion preventing or restricting element comprises a sleeve made from an elastically deformable material, extending said distal end of the sheath, having, in the non-inflated state of said first balloon, a conical shape converging radially, i.e. extending from said distal end of the sheath to said first balloon, and expanding radially during the inflation of said first balloon, to adopt a flared shape, radially divergent.

This radial expansion generates a radial elastic return force of the material making up said sleeve, favorable to obtaining an effective removal movement of the sheath.

This expansion preventing or restricting element can also comprise a ring made from a material not radially stretchable, fixed at the distal end of the sheath. This ring can be made from a material with a low coefficient of friction, for example a metal, for the same reasons as indicated above. The ring can also form a radiopaque element.

The invention will be well understood, and other features and advantages thereof will appear, in reference to the appended diagrammatic drawing, showing, as nonlimiting examples, several possible embodiments of the concerned delivery system.
Figure 1 is a view in longitudinal cross-section according to a first embodiment, when it is engaged in a bodily conduit, before deployment of the stent comprised by that system;
figure 2 is a view similar to Figure 1, at the beginning of the deployment procedure of the stent;
figure 3 is a view similar to Figure 1, during the deployment procedure of the stent;
figure 4 is a view similar to Figure 1, at the end of the procedure for deploying the stent;
figure 5 is a view similar to Figure 4, according to another embodiment;
figure 6 is a view similar to Figure 4, according to still another embodiment;
figure 7 is a cross-sectional view similar to Figure 1, according to still another embodiment;
figure 8 is a view similar to Figure 7 of this same embodiment, at the beginning of the procedure for deploying the stent;
figure 9 is a cross-sectional view of the system similar to Figure 1, according to still another embodiment;
figure 10 is a cross-sectional view similar to Figure 1, according to still another embodiment;
figure 11 is a cross-sectional view of this same embodiment, at the beginning of the procedure for deploying the stent;
figure 12 is a cross-sectional view of the system similar to Figure 1, according to still another embodiment;
figures 13 to 15 are transverse cross-sectional views thereof, according to three possible embodiments of a balloon comprised by said system;
figure 16 is a cross-sectional view of the system similar to Figure 1, according to still another embodiment;
figure 17 is a cross-sectional view of the system similar to Figure 1, according to still another embodiment;
figure 18 is a cross-sectional view of the system similar to Figure 1, according to still another embodiment;
figure 19 is a perspective view of a sliding element in the form of a sleeve, comprised by the system shown in Figure 18;
figure 20 is a cross-sectional view of the system similar to Figure 1, according to still another embodiment, in the uninflated state of a balloon comprised by said system;
figure 21 is a view of the system shown in Figure 20, at the beginning of inflation of said balloon, and
figure 22 is a side view of another embodiment of said balloon.

For simplification, the parts or elements of one embodiment that are found identically or similarly in another embodiment will be designated using the same numerical references.

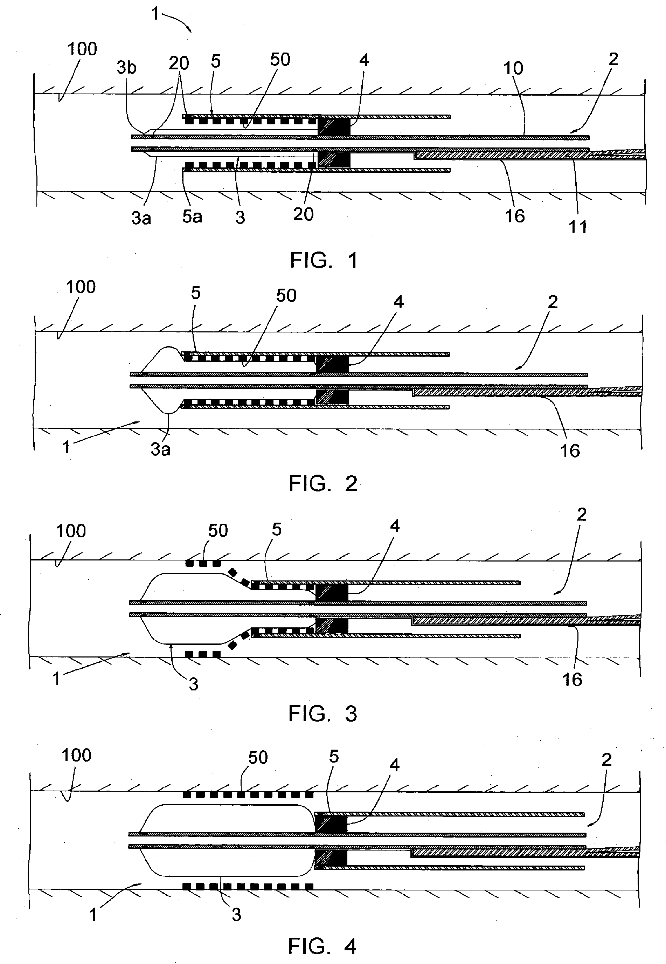

Figures 1 to 4 show a system 1 for delivering a stent 50, engaged in a bodily conduit 100.

The stent 50 is, as is well known, intended to reestablish the caliber of the bodily conduit 100, particularly at a stenosis formed by atheromatous plaques (not shown). It is formed by a deployable tubular framework, with an mesh structure, which can adopt either the radially contracted state shown in Figure 1, which allows it to be inserted into the bodily conduit 100, or the radially expanded state shown in Figure 4, in which it is implanted and reestablishes the caliber of the bodily conduit 100. The stent 50 can be made from an elastic material making it self-expanding, for example an alloy of nickel and titanium, or can be made from a material making it necessary to perform, in whole or in part, a forced expansion.

The system 1 comprises an inner support 2 comprising the stent 50, an inflatable balloon 3, a stop 4 and an outer sheath 5.

The inner support 2 comprises a distal tubular portion 10 connected to a proximal portion 11.

The distal portion 10 comprises the balloon 3 and the stop 4, fixed thereto.

The proximal portion 11 has a sufficient length to protrude to the outside of the patient when the distal portion 10 is found at the implantation site of the stent 50, and thus makes it possible to move the system 1 relative to the bodily conduit 100.

The balloon 3 is formed by a flexible enclosure engaged around a distal part of the distal portion 10. On the distal side, this flexible enclosure is sealably fixed to the distal portion 10, on the entire periphery of said distal portion, at one end 3b; on the proximal side, the flexible enclosure is fixed to a radially inner zone of the distal surface of the stop 4, as shown, or to the distal portion 10, near said stop. In the inflated state, the balloon 3 has a transverse section larger than that of the sheath 5; in the example embodiment shown in Figures 1 to 4, it is cylindrical, and has a transverse section smaller than that of the bodily conduit 100.

The balloon 3 has a length greater than that of the stent 50 and thus comprises a distal portion 3a, adjacent to the distal end 3b, which protrudes past the distal end 5a of the sheath 5, on the distal side thereof, as particularly visible in Figure 1.

This balloon 3 is supplied with pressurized fluid from outside the patient, through a conduit 16 running along the support 2 and passing between the distal portion 10 and the stop 4.

The latter is formed by a thick washer secured to the distal tubular portion 10, the diameter of which is equal to or slightly larger than the inner diameter of the sheath 5, so as to allow sliding of that sheath 5 thereon but with friction. The proximal end of the stent 50 bears against the distal surface of said stop 4.

The sheath 5 is engaged around the stent 50, whereof it ensures, in the distal position shown in Figure 1, the maintenance in the radially contracted state. It is engaged on the stop 4 and, in the illustrated example, extends over the proximal side of said stop 4, over a length in the vicinity of two thirds that of the stent 50. This sheath 5 therefore does not extend along the proximal portion 11 of the support 2 and is not intended to be slidingly actuated from outside the patient, like a traditional counterpart sheath, but to be actuated by inflating the balloon 3, as described below.

It appears that, in the distal position of the sheath 5, overlapping the stent 50, the distal end 5a of the sheath 5 is situated back from said distal end 3b, along said longitudinal axis of the support 2, in the proximal direction, and is situated opposite said distal portion 3a, so that this distal portion 3a protrudes partially from said distal end 5a of the sheath 5, on the distal side of said end.

In practice, the system 1 is inserted and is slid in the bodily conduit 100 in the state shown in Figure 1. The maintenance of the sheath 5 in the distal overlapping position of the stent 50 is ensured owing to the friction existing between the stop 4 and the sheath 5.

The arrival of the distal portion of the system 1 at the implantation site is detected using radiopaque markers 20 comprised by the support 2 and the sheath 5, in particular, in the example shown in Figure 1, a marker 20 situated on the portion 10 at the distal end 3b of the balloon 3, a marker 20 situated on the portion 10 at the proximal end of the balloon 3, and a marker 20 situated at the distal end of the sheath 5.

The balloon 3 is then supplied with inflating fluid, which first inflates the distal portion 3a thereof and then creates an inflation wave moving in the proximal direction. This inflation wave causes the balloon 3 to come into contact with the distal end 5a of the sheath 5 (see Figure 2), then causes that sheath to slide along the wall of said balloon 3, which makes it possible to remove the sheath 5 and therefore to gradually release the stent 50 (see Figures 3 and 4).

At the beginning of this removal, the distal end of the stent 50 is maintained by the distal portion 3a of the balloon 3, which prevents any risk of "jumping" of the stent 50 relative to the support 2. The bearing of the proximal end of the stent 50 against the stop 4 prevents the risk of removal of the stent 50 at the same time as the sheath 5.

The removal of the sheath 5 is therefore done without any pulling force having been exerted on this sheath from outside the patient, which prevents the risk of movement of the system 1, and therefore of the stent 50, relative to the implantation site. On the contrary, the inflation of the distal portion 3a of the balloon immediately presses the distal end of the stent 50 against the wall of the bodily conduit 100 as of the beginning of removal of the sheath 5, and therefore performs a certain axial immobilization of that stent 50 relative to said conduit 100 as of this beginning of removal.

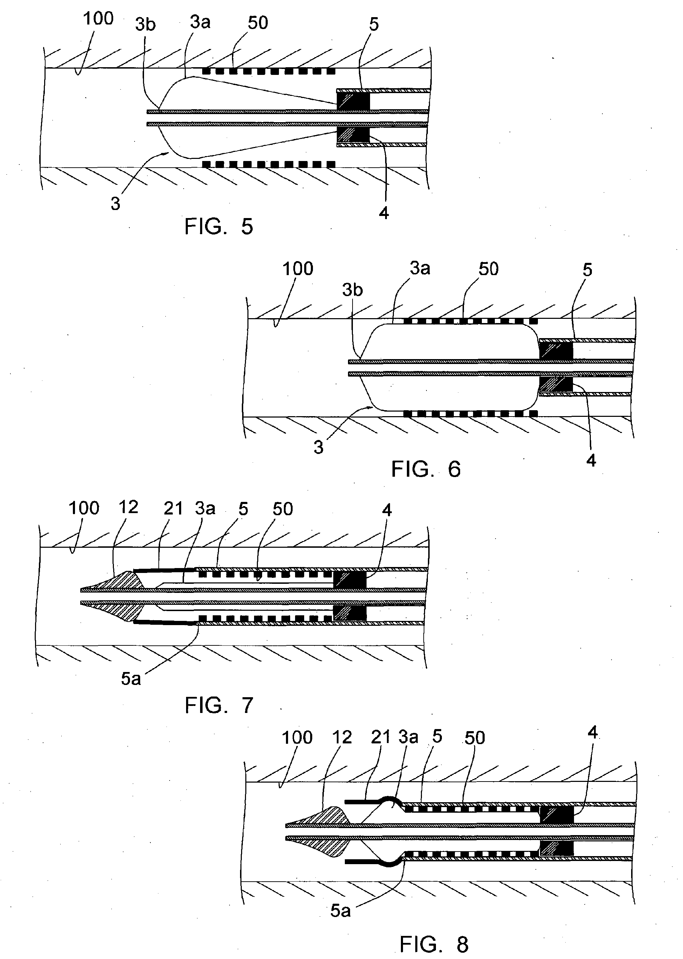

Figure 5 shows another embodiment of the system 1, in which the balloon 3 is conical, with the exception of its distal surface, which is rounded.

Figure 6 shows an embodiment of the system 1 that is practically identical to that previously described, except that the balloon 3 has a larger diameter, slightly smaller than that of the bodily conduit 100. Such a balloon 3 is used in particular when it involves performing a forced deployment of the stent 50.

Figures 7 and 8 show another embodiment of the system 1, in which said distal portion 10 comprises, at its distal end, a widened and rounded end piece 12 (commonly called "tip") and the sheath 5 comprises a sleeve 21 extending its distal end, which is dimensioned so as to extend as far as the end piece or tip 12. This sleeve 21 is made from a material more flexible than that of the sheath 5, not offering resistance to the inflation of the distal portion 3a of the balloon 3. The end piece 12 favors the insertion and sliding of the system 1 in the conduit 100, and the sleeve 21 allows that system to have a substantially smooth and continuous outer wall between the end piece 12 and the sheath 5 (see Figure 7).

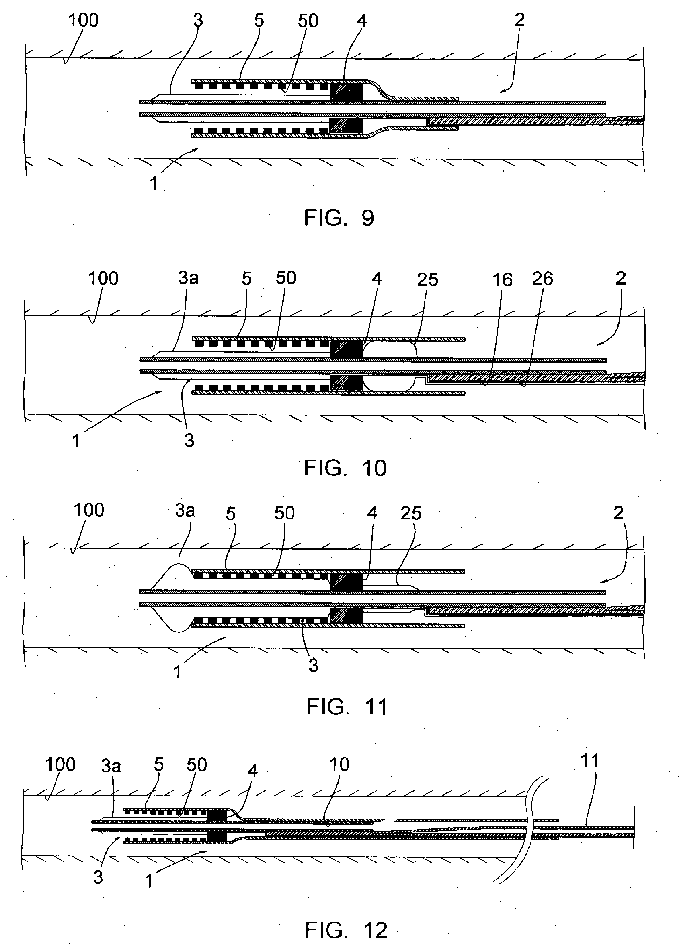

Figure 9 shows another embodiment of the system 1, in which the sheath 5 bends on the proximal side, beyond the stop 4, and is connected to the support 2.

This connection favors the maintenance of the sheath 5 relative to the support 2, in said distal position. The sheath 5, during the removal thereof, folds on the proximal side at its bend.

Figures 10 and 11 show another embodiment of the system 1, in which this system comprises a second inflatable balloon 25, fixed to the support 2 close to the stop 4, on the proximal side thereof. This second balloon 25 is supplied with inflation fluid, from outside the patient, through a conduit 26 separate from the supply conduit 16 of the balloon 3. As shown in Figure 10, it is capable, in the inflated state, of being pressed against the inner surface of the sheath 5 so as to generate friction therewith, immobilizing the latter in the axial direction relative to the support 2. It consequently forms locking means for locking the sheath 5 in its distal position, preventing any risk of removal of that sheath during the insertion and sliding of the system 1 in the bodily conduit 100.

In practice, this second balloon 25 is in the inflated state during the insertion and sliding of the system 1 in the bodily conduit (see Figure 10), thereby locking the sheath 5 in its distal position, and is deflated just before the deployment operation of the stent 50, so as to free the removal of the sheath 5 (see Figure 11).

Figure 12 shows an alternative in which the sheath 5 has a length such that it is capable of extending outside the patient when the stent 50 is opposite the implantation site. Its immobilization can therefore be done and controlled from outside the patient, using suitable immobilization means, which are released before beginning the inflation of the balloon 3.

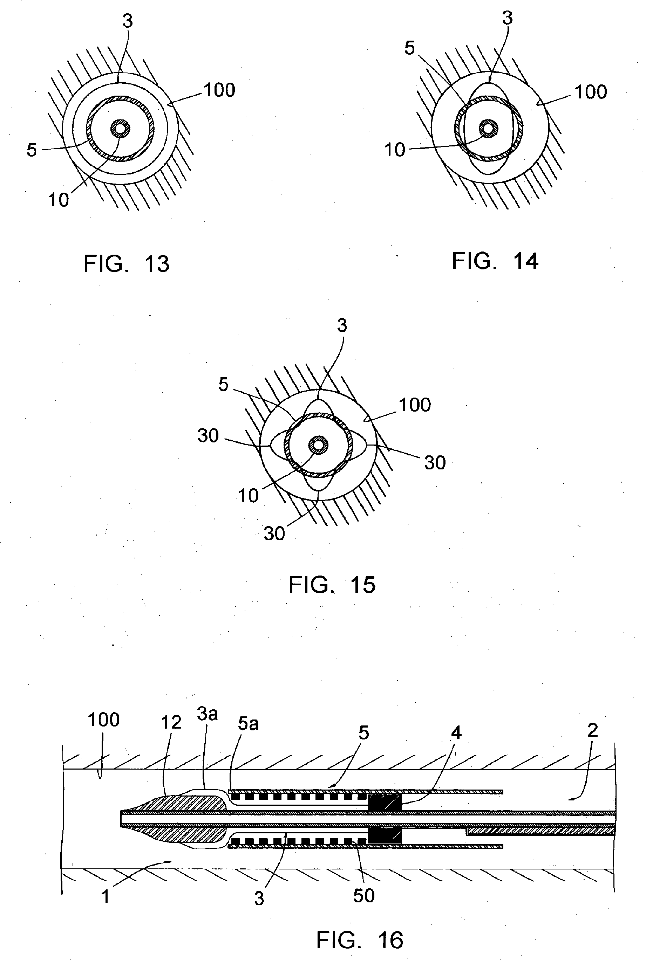

Figure 13 shows the most frequent case of a balloon 3 assuming a circular shape in transverse section. Figure 14 shows that it can also have an oval shape in transverse cross-section, and Figure 15 that it can also have one or several longitudinal ribs 30, in particular four equidistant longitudinal ribs 30.

Figure 16 shows another embodiment of the system 1, in which the distal portion 3a of the balloon 3 is dimensioned to surround a proximal part of the distal end piece 12 and is, on the distal side, connected thereto, over its entire periphery. This distal portion 3a is also shaped so as to extend, in the uninflated state, close to the distal end 5a of the sheath 5.

The balloon 3, thus shaped, allows the system 1 to have a substantially smooth and continuous outer wall between the end piece 12 and the sheath 5.

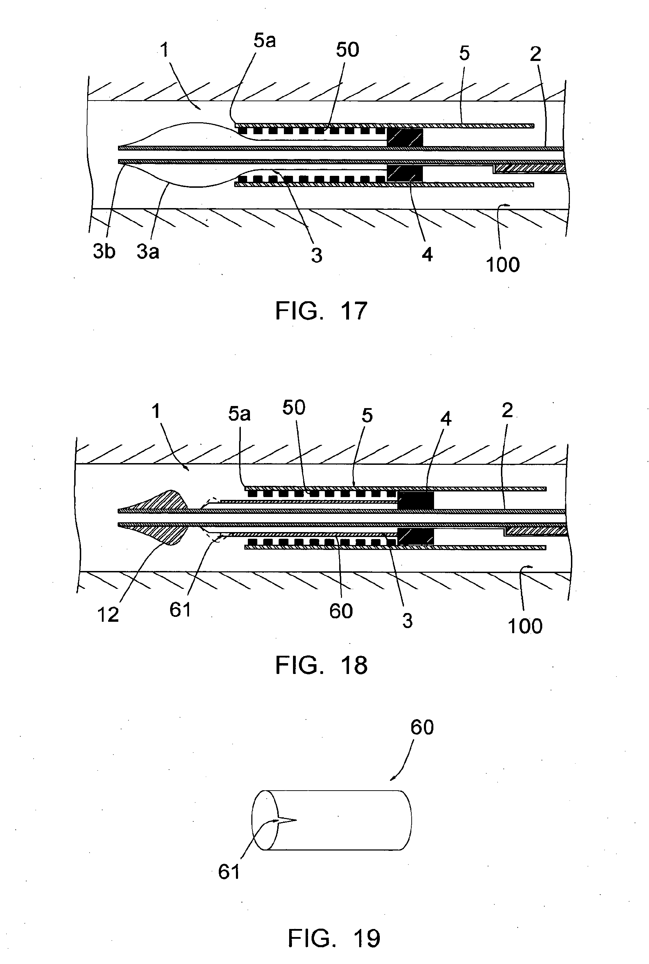

Figure 17 shows another embodiment of the system 1, in which the balloon 3 has, in the uninflated state or the folded state illustrated, a distal portion 3a having a transverse section larger than that of the proximal portion of that balloon 3, this distal portion 3a having a more or less ovoid or hernial shape.

Figure 18 shows still another embodiment of the system 1, in which the system includes a proximal inflation restricting element 60. This element 60 makes it possible to ensure that the inflation of the balloon 3 starts at the distal portion 3a of said balloon and progresses in the proximal direction, and to thereby prevent early proximal inflation from occurring, i.e. proximal inflation occurring at the same time as the distal inflation.

In the example shown in this Figure 18, this restricting element 60 is in the form of a sleeve made from PTFE (polytetrafluoroethylene), surrounding the balloon 3 in an adjusted manner, having a distal end that protrudes past the distal end 5a of the sheath 5 and that is situated back from the distal end 3b of the balloon 3 in the proximal direction. This sleeve can be torn longitudinally under the effect of the inflation of the balloon 3, so as not to hinder that inflation.

The inflation of the balloon 3 is thus done from said distal end 3b and progresses in the proximal direction, the proximal portion of the balloon 3 not being able to inflate, being maintained by the sleeve; this inflation causes the progressive tearing of the sleeve from said distal end of the sleeve, which allows the inflation to continue, in the proximal direction.

As shown in Figure 19, the sleeve can comprise a longitudinal notch 61 formed from its distal end, constituting a beginning of longitudinal tearing.

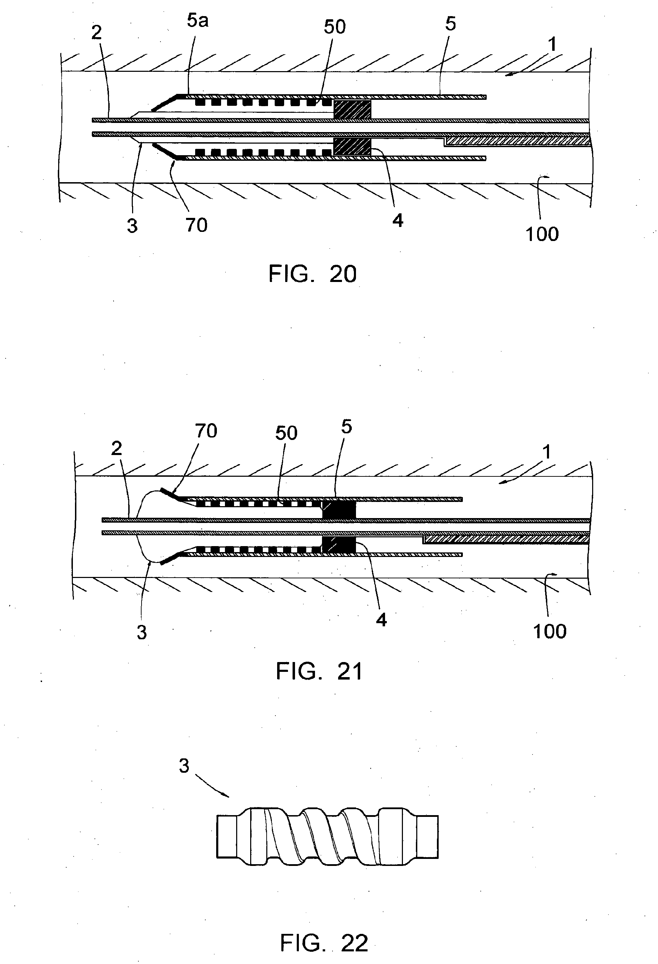

Figures 20 and 21 show still another embodiment of the system 1, in which said system includes an element 70 for preventing or restricting the radial expansion of the sheath 5, also resulting in preventing an early proximal inflation of the balloon 3 from occurring.

In the example embodiment shown in Figures 20 and 21, this element 70 comprises a sleeve made from an elastically deformable material, extending the distal end 5a of the sheath 5. This element 70 has, in the non-inflated state of the balloon 3, a radially convergent conical shape (see Figure 20), i.e. extending from the distal end 5a to said first balloon, and expanding radially during the inflation of said balloon 3, to adopt a flared shape, radially divergent (see Figure 21).

This radial expansion generates a radial elastic return force of the material making up said sleeve, favorable to obtaining an effective withdrawal movement of the sheath 5.

The element 70 can also comprise a ring made from a material that is not radially stretchable, fixed at the distal end 5a of the sheath 5.

Figure 22 shows that the balloon 3 can also assume, in the inflated state, a helical groove 80, the purpose of which is to reduce the force exerted radially by the balloon 3 on the sheath 5 as well as the friction existing between this balloon 3 and the stent 50.

As appears from the preceding, the invention provides a system for delivering a stent having, relative to its counterpart systems of the prior art, the decisive advantage of allowing precise positioning of the stent in the bodily conduit to be treated.

The invention was described above in reference to embodiments provided as examples. It is of course not limited to these embodiments, but extends to all other embodiments covered by the appended claims.

## Claims

1. Stent (50) delivery system (1) comprising:
- a stent (50) having a proximal end;
- a support (2) comprising the stent (50) engaged thereon, having a longitudinal axis;
- a sheath (5) having a distal end (5a), which is capable to be slid relative to said support (2) between a distal overlap position of the stent (50) and a proximal release position for the deployment of the stent (50), and
- a first inflatable balloon (3) situated between said support (2) and the stent (50), comprising a distal portion (3a) that protrudes past the distal end (5a) of the sheath, on the distal side thereof, when the latter is in said distal position;
**characterized in that**:
- said support (2) is equipped with a stop (4) against which the proximal end of the stent (50) is applied;
- said sheath (5) is capable to be slid relative to said stop (4), and the distal end (5a) of this sheath (5) is, in said distal overlap position of the stent (50), situated back from the distal end (3b) of said first inflatable balloon (3), along said longitudinal axis of the support (2), in the proximal direction, and is situated opposite said distal portion (3a); and
- said first inflatable balloon (3) has, in the inflated state, over all the length thereof or over part of the length thereof, on the distal side, a transverse section larger than or equal to that of the sheath (5).

2. System (1) according to claim 1, **characterized in that** it includes locking means (5, 25) that are able to be actuated between a locking position and an unlocking position, these locking means (5, 25) being able, in the locking position, to immobilize the sheath (5) in said distal overlap position and, in the unlocking position, to release the sliding of the sheath (5).

3. System (1) according to claim 2, **characterized in that** the locking means comprise a sheath (5) having a length such that said sheath (5) extends outside the patient when the stent is located opposite the implantation site, and means for immobilizing said sheath (5) relative to said support (2), capable of being controlled from outside the patient.

4. System (1) according to claim 2, **characterized in that** it comprises a second inflatable balloon (25), fixed in position relative to said support (2) and situated inside the sheath, the inflation of which corresponds to said locking position and the deflation of which corresponds to said unlocking position; in the inflated state, this second balloon (25) is pressed against the inner surface of the sheath (5) so as to generate friction therewith, immobilizing the latter in the axial direction relative to said support (2), and, in the deflated state, this second balloon (25) is not pressed against the inner surface of the sheath (5) so as to allow the sliding of the latter.

5. System (1) according to claim 4, **characterized in that** the said second balloon (25) is situated close to said stop (4), on the proximal side thereof.

6. System (1) according to claim 2, **characterized in that** locking means comprise a second inflatable balloon fixed in position relative to said support and the inflation of which corresponds to said locking position while the deflation thereof corresponds to said unlocking position; this second balloon is situated outside the sheath, on the proximal side of a proximal end of said sheath; the second balloon has, in the inflated state, a diameter larger than that of the sheath, so that this second balloon forms a stop against which the proximal end of the sheath bears so as to block the sliding of the sheath; in the deflated state, this second balloon has a diameter smaller than that of the sheath, so that this second balloon does not form a stop for said proximal end of the sheath and does not hinder the sliding of the sheath.

7. System (1) according to claims 1 to 6, **characterized in that** the distance separating the distal end of said balloon (3) and the distal end (5a) of the sheath (5) when the latter is in the distal position, is comprised between one and two times the diameter of the sheath (5).

8. System (1) according to claims 1 to 7, **characterized in that** said first balloon (3) has a cylindrical shape, with a diameter at least equal to that of the sheath (5).

9. System (1) according to claims 1 to 8, **characterized in that** said first balloon (3) has a conical shape, whereof the distal portion (3a), with the larger diameter, has a diameter at least equal to that of the sheath (5).

10. System (1) according to claims 1 to 7, **characterized in that** said first balloon (3) has an oval transverse section.

11. System (1) according to claims 1 to 7, **characterized in that** said first balloon (3) has one or more longitudinal ribs (30), in particular four equidistant longitudinal ribs (30).

12. System (1) according to claims 1 to 11, **characterized in that** said support (2) comprises a widened distal end piece (12), capable of favoring the progression of the system (1) in a bodily conduit (100), and the sheath (5) comprises a sleeve (21) extending its distal end (5a), which is dimensioned so as to extend to said end piece (12), this sleeve (21) being made from a material more flexible than that of the sheath (5), not offering resistance to the inflation of said distal portion (3a) of said first balloon (3), or a low resistance to that inflation.

13. System (1) according to claims 1 to 11, **characterized in that** said support (2) comprises a widened distal end piece (12), capable of favoring the progression of the system (1) in a bodily conduit (100), and said distal portion (3a) of the first balloon (3) is shaped so as to surround a proximal portion of that end piece (12) and, in the non-inflated state, to extend close to the distal end (5a) of the sheath (5).

14. System (1) according to claims 1 to 13, **characterized in that** it includes a proximal inflation restricting element (60), making it possible to ensure that the inflation of said first balloon (3) starts at said distal portion of the balloon (3) and progresses in the proximal direction.

15. System (1) according to claim 14, **characterized in that** the restriction element (60) is in the form of a sleeve surrounding said balloon (3) in an adjusted manner, one distal end of said sleeve protruding past the distal end (5a) of the sheath (5) and being situated withdrawn from the distal end (3b) of said first balloon (3) in the proximal direction, and this sleeve being longitudinally tearable under the effect of the inflation of this first balloon (3), so as not to hinder the inflation.

16. System (1) according to claim 15, **characterized in that** said sleeve is made from a material with a low coefficient of friction, in particular PTFE (polytetrafluoroethylene).

17. System (1) according to claim 15, **characterized in that** said sleeve comprises a longitudinal notch (61) arranged from its distal end, constituting a beginning of longitudinal tearing.

18. System (1) according to claims 1 to 17, **characterized in that** it includes an element (70) for preventing or restricting the radial expansion of the sheath (5).

19. System (1) according to claim 18, **characterized in that** the expansion preventing or restricting element (70) comprises a sleeve made from an elastically deformable material, extending said distal end (5a) of the sheath (5), having, in the non-inflated state of said first balloon (3), a conical shape converging radially, i.e. extending from said distal end (5a) of the sheath (5) to said first balloon (3), and expanding radially during the inflation of said first balloon (3), to adopt a flared shape, radially divergent.

20. System (1) according to claims 18 to 19, **characterized in that** the expansion preventing or restricting element (70) can comprise a ring made from a material not radially stretchable, fixed at the distal end (5a) of the sheath (5).

## Patentansprüche

1. Stent (50) Abgabesystem (1), umfassend:
- Einen Stent (50) mit einem proximalen Ende;
- Einen Träger (2) mit dem Stent (50) darauf in Eingriff gebracht, mit einer Längsachse;
- eine Hülle (5) mit einem distalen Ende (5a), angepasst, um in Bezug verschoben werden, um den Träger (2) zwischen einer distalen Abdeckstellung des Stents (50) und eine proximale Position der Freigabe der Entfaltung des Stents (50), und
- Einen ersten aufblasbaren Ballon (3), die sich zwischen der Unterlage (2) und der Stent (50), mit einem distalen Abschnitt (3a), die über das distale Ende (5a) der Hülle hinausragt, auf der distalen Seite davon, wenn das Letzteres ist in der distalen Position befindet;
**dadurch gekennzeichnet, dass**:
- der Halter (2) mit einem Anschlag (4), gegen die sich das proximale Ende des Stents (50) aufgebracht ist ausgestattet;
- die Hülle (5) angepasst ist, um in Bezug verschoben werden, um den Anschlag (4), und das distale Ende (5a) der Hülle (5) in der distalen Position, in welcher der Stent (50), von dem distalen Ende zurückversetzt (3b) der ersten aufblasbaren Ballon (3), entlang der Längsachse des Trägers (2) in proximaler Richtung und ist gegenüber dem genannten distalen Abschnitts (3a); und
- der erste aufblasbare Ballon (3) im aufgeblasenen Zustand über die ganze Länge desselben oder über einen Teil der Länge desselben, auf der distalen Seite ein Querschnitt größer oder gleich dem von der Hülle (5).

2. System (1) nach Anspruch 1, **dadurch gekennzeichnet, dass** sie betätigbare Verriegelungsmittel (5, 25) zwischen einer Verriegelungsposition und einer Entriegelungsstellung umfasst, diese Verriegelungsmittel (5, 25) sind in der Lage, in der Verriegelungsposition, um die Hülle (5) in die distale Übergangsstellung zu immobilisieren und in der Entriegelungsstellung zur Freigabe der Gleitbewegung der Hülle (5).

3. System (1) nach Anspruch 2, **dadurch gekennzeichnet, dass** die Verriegelungsmittel umfassen eine Hülle (5) mit einer Länge derart, dass die Hülle (5) sich außerhalb des Patienten, wenn der Stent (50) vor der Stelle der Implantation, und Mittel zum Immobilisieren die Hülle (5) gegenüber dem Träger (2), in der Lage, von außerhalb des Patienten gesteuert werden.

4. System (1) nach Anspruch 2, **dadurch gekennzeichnet, dass** sie einen zweiten aufblasbaren Ballon (25), in seiner Position relativ zu dem Träger (2) und innerhalb der Hülle angeordnet aufweist, wobei Schwellung entspricht der Verriegelungsposition und Entleeren entspricht der Entriegelungsstellung; wenn er aufgeblasen ist, der zweite Ballon (25) kommt in Anlage an der Innenfläche der Hülle (5), um die Reibung damit die Immobilisierung des letzteren in der axialen Richtung relativ zu erzeugen, um den Träger (2) und im entleerten Zustand, der zweite Ballon (25) nicht in Anlage gegen die Innenfläche der Hülle (5) zu kommen, um das Gleiten des letzteren zu ermöglichen.

5. System (1) nach Anspruch 4, **dadurch gekennzeichnet, dass** der zweite Ballon (25) in der Nähe befindet, der Anschlag (4), der proximalen Seite davon.

6. System (1) nach Anspruch 2, **dadurch gekennzeichnet, dass** die Verriegelungseinrichtung eine zweite aufblasbare Ballon in einer festen Position relativ zu dem Träger, und dessen Quellung entspricht der Verriegelungsposition und Deflation entspricht der Entriegelungsstellung; der zweite Ballon außerhalb der Hülle proximal von einem proximalen Ende der Hülle befindet; der zweite Ballon im aufgeblasenen Zustand einen Durchmesser größer als derjenige der Hülle, so dass sie einen Anschlag, gegen den Halter das proximale Ende der Hülle zu blockieren das Gleiten der Hülle bildet; in dem entleerten Zustand weist diese zweite Ballon einen Durchmesser kleiner als derjenige der Hülle, so dass diese zweite Ballon nicht einen Anschlag für das proximale Ende der Hülle zu bilden, und behindert nicht das Gleiten der Ummantelung.

7. System (1) nach den Ansprüchen 1 bis 6, **dadurch gekennzeichnet, dass** der Abstand zwischen dem distalen Ende des Ballons (3) und das distale Ende (5a) der Hülle (5), wenn sich letztere in der distalen Position ist umfasste zwischen ein und zwei mal der Durchmesser der Hülle (5).

8. - System (1) nach den Ansprüchen 1 bis 7, **dadurch gekennzeichnet, dass** der erste Ballon (3) eine zylindrische Form mit einem Durchmesser wenigstens gleich dem der Hülle (5).

9. System (1) nach den Ansprüchen 1 bis 8, **dadurch gekennzeichnet, dass** der erste Ballon (3) eine konische Form besitzt, wovon sich der distale Abschnitt (3a) mit dem größeren Durchmesser hat, deren Durchmesser mindestens gleich dem der Hülle (5).

10. System (1) nach den Ansprüchen 1 bis 7, **dadurch gekennzeichnet, dass** der erste Ballon (3) einen ovalen Querschnitt aufweist.

11. System (1) nach den Ansprüchen 1 bis 7, **dadurch gekennzeichnet, dass** der erste Ballon (3) umfasst eine oder mehrere Längsrippen (30), insbesondere vier äquidistante Längsrippen (30).

12. System (1) nach den Ansprüchen 1 bis 11, **dadurch gekennzeichnet, dass** der Träger (2) einen verbreiterten distalen Endstück (12), fähig eine Begünstigung des Fortschreitens des Systems (1) in einem Körperkanal (100), und die **dadurch gekennzeichnet** Hülle (5) eine Hülse (21), der sich sein distales Ende (5a), die so dimensioniert ist, um das Endstück (12) zu erstrecken, diese Hülse (21) ist einem flexibleren Material als das der Hülle gemacht (5) so dass bietet keinen Widerstand zum Aufblähen des distalen Abschnitts (3a) des ersten Ballons (3) oder eine geringe Beständigkeit gegenüber Quellung.

13. System (1) nach den Ansprüchen 1 bis 11, **dadurch gekennzeichnet, dass** der Träger (2) einen verbreiterten distalen Endstück (12), fähig eine Begünstigung des Fortschreitens des Systems (1) in einem Körperkanal (100) und das distalen Abschnitts (3a) des ersten Ballons (3) so geformt ist, einen proximalen Abschnitt der genannten Endstück (12) zu umgeben, und in dem nicht-aufgeblasenen Zustand ist, um bis nahe zum distalen Ende (5a) der Hülle (5).

14. System (1) nach den Ansprüchen 1 bis 13, **dadurch gekennzeichnet, dass** sie ein Element (60) die Begrenzung der proximalen Inflations umfasst, dies stellt sicher, dass das Aufblasen des ersten Ballons (3), von dem distalen Abschnitt des Ballons (3), in der proximalen Richtung bewegt.

15. System (1) nach Anspruch 14, **dadurch gekennzeichnet, dass** das Beschränkungselement (60) ist in der Form einer umgebenden Hülse des Ballons (3) in einer angepassten Weise einem distalen Ende der Hülse über das distale Ende vorsteht (5a) der Hülle (5) erstreckt und vom distalen Ende (3b) des ersten Ballons (3) in proximaler Richtung, und diese Hülse längs reißbar unter der Wirkung des Aufblasens des ersten Ballons (3), so gelegen zurückgezogen nicht um das Aufblasen zu verhindern.

16. System (1) nach Anspruch 15, **dadurch gekennzeichnet, dass** die Hülse aus einem Material mit einem niedrigen Reibungskoeffizienten, insbesondere aus PTFE (Polytetrafluorethylen).

17. System (1) nach Anspruch 15 oder Anspruch 16, **dadurch gekennzeichnet, dass** die Hülse weist einen von einem distalen Ende davon ausgebildet Längsnut (61), eine Längsreißinitiierungs bildet.

18. System (1) nach den Ansprüchen 1 bis 17, **dadurch gekennzeichnet, dass** sie ein Element (70) zum Verhindern oder Einschränken der radialen Expansion der Hülle (5) aufweist.

19. System (1) nach Anspruch 18, **dadurch gekennzeichnet, dass** die Verhinderungselement (70) oder zur Beschränkung der radialen Ausdehnung der Hülle (5) eine Hülse (70) aus einem elastisch verformbaren Material, der sich von dem distalen Ende (5a) der Hülle (5), und in dem Zustand des Nicht-Aufblasen des ersten Ballons (3), eine konische Form radial konvergierenden das heißt, die sich von dem Ende distal (5a) der Hülle (5) zu dem ersten Ballon (3) und radialen Aufweiten beim Aufblasen des ersten Ballons (3), um eine aufgeweitete Form annehmen, radial Abfackeln.

20. System (1) nach den Ansprüchen 18 oder Ansprüchen 19, **dadurch gekennzeichnet, dass** der Verhinderungselement (70) oder zur Beschränkung der radialen Ausdehnung der Hülle (5) umfasst einen Ring aus einem Material, das nicht radial dehnbar, am distalen Ende (5a) der Hülle (5) befestigt ist.

## Revendications

1. Système (1) de délivrance d'un stent (50), comprenant :
- un stent (50) ayant une extrémité proximale ;
- un support (2) comportant le stent (50) engagé sur lui, ayant un axe longitudinal ;
- une gaine (5) ayant une extrémité distale (5a), apte à être coulissée par rapport audit support (2) entre une position distale de recouvrement du stent (50) et une position proximale de libération du déploiement du stent (50), et
- un premier ballonnet gonflable (3) situé entre ledit support (2) et le stent (50), comprenant une portion distale (3a) dépassant de ladite extrémité distale (5a) de la gaine, sur le côté distal de celle-ci, lorsque cette dernière est dans ladite position distale ;
**caractérisé en ce que**:
- ledit support (2) est équipé d'une butée (4) contre laquelle l'extrémité proximale du stent (50) est appliquée ;
- ladite gaine (5) est apte à être coulissée par rapport à ladite butée (4), et l'extrémité distale (5a) de cette gaine (5) est, dans ladite position distale de recouvrement du stent (50), située en retrait de l'extrémité distale (3b) dudit premier ballonnet gonflable (3), le long dudit axe longitudinal du support (2), dans la direction proximale, et est située à l'opposé de ladite partie distale (3a) ; et
- ledit premier ballonnet gonflable (3) présente, à l'état gonflé, sur toute sa longueur ou sur une partie de sa longueur, sur le côté distal, une section transversale supérieure ou égale à celle de la gaine (5).

2. Système (1) selon la revendication 1, **caractérisé en ce qu'**il inclut des moyens de verrouillage (5 ; 25) actionnables entre une position de verrouillage et une position de déverrouillage, ces moyens de verrouillage (5 ; 25) étant aptes, en position de verrouillage, à immobiliser la gaine (5) dans ladite position distale de recouvrement, et, en position de déverrouillage, à libérer le coulissement de cette gaine (5).

3. Système (1) selon la revendication 2, **caractérisé en ce que** les moyens de verrouillage comprennent une gaine (5) ayant une longueur telle qu'elle est apte à s'étendre jusqu'en dehors du patient lorsque le stent (50) se trouve en regard du site d'implantation, et des moyens d'immobilisation de la gaine (5) par rapport audit support (2), aptes à être commandés depuis l'extérieur du patient.

4. Système (1) selon la revendication 2, **caractérisé en ce qu'**il comprend un deuxième ballonnet gonflable (25), fixe en position par rapport audit support (2) et situé à l'intérieur de la gaine, dont le gonflement correspond à ladite position de verrouillage et le dégonflement correspond à ladite position de déverrouillage ; à l'état gonflé, ce deuxième ballonnet (25) vient en application contre la face interne de la gaine (5) de manière à générer des frottements avec celle-ci immobilisant cette dernière dans le sens axial par rapport audit support (2), et, à l'état dégonflé, ce deuxième ballonnet (25) ne vient pas en application contre la face interne de la gaine (5) de manière à autoriser le coulissement de cette dernière.

5. Système (1) selon la revendication 4, **caractérisé en ce que** ledit deuxième ballonnet (25) est situé à proximité de ladite butée (4), du côté proximal de celle-ci.

6. Système (1) selon la revendication 2, **caractérisé en ce que** les moyens de verrouillage comprennent un deuxième ballonnet gonflable fixe en position par rapport audit support et dont le gonflement correspond à ladite position de verrouillage tandis que le dégonflement correspond à ladite position de déverrouillage ; ce deuxième ballonnet est situé à l'extérieur de la gaine, sur le côté proximal d'une extrémité proximale de cette gaine ; le deuxième ballonnet a, à l'état gonflé, un diamètre supérieur à celui de la gaine, de sorte qu'il forme une butée contre laquelle porte l'extrémité proximale de la gaine afin de bloquer le coulissement de la gaine ; à l'état dégonflé, ce deuxième ballonnet a un diamètre inférieur à celui de la gaine, de sorte qu'il ne forme pas une butée pour ladite extrémité proximale de la gaine et ne fait donc pas obstacle au coulissement de la gaine.

7. Système (1) selon l'une des revendications 1 à 6, **caractérisé en ce que** le déport distal de ladite portion distale (3a) dudit premier ballonnet (3), c'est-à-dire la distance séparant l'extrémité distale de ce ballonnet (3) et l'extrémité distale (5a) de la gaine (5) lorsque cette dernière est en position distale, est compris entre une fois et deux fois le diamètre de la gaine (5).

8. Système (1) selon l'une des revendications 1 à 7, **caractérisé en ce que** ledit premier ballonnet (3) a une forme cylindrique, de diamètre au moins égal à celui de la gaine (5).

9. Système (1) selon l'une des revendications 1 à 8, **caractérisé en ce que** ledit premier ballonnet (3) a une forme conique, dont la portion distale (3a), de plus grand diamètre, a un diamètre au moins égal à celui de la gaine (5).

10. Système (1) selon l'une des revendications 1 à 7, **caractérisé en ce que** ledit premier ballonnet (3) a une section transversale ovale.

11. Système (1) selon l'une des revendications 1 à 7, **caractérisé en ce que** ledit premier ballonnet (3) inclut une ou plusieurs nervures longitudinales (30), notamment quatre nervures longitudinales (30) équidistantes.

12. Système (1) selon l'une des revendications 1 à 11, **caractérisé en ce que** ledit support (2) comprend un embout distal élargi (12), apte à favoriser la progression du système (1) dans un conduit corporel (100), et **en ce que** la gaine (5) comporte un manchon (21) prolongeant son extrémité distale (5a), qui est dimensionné de manière à s'étendre jusqu'audit embout (12), ce manchon (21) étant en un matériau plus souple que celui de la gaine (5), n'offrant pas de résistance au gonflement de ladite portion distale (3a) dudit premier ballonnet (3), ou une faible résistance à ce gonflement.

13. Système (1) selon l'une des revendications 1 à 11, **caractérisé en ce que** ledit support (2) comprend un embout distal élargi (12), apte à favoriser la progression du système (1) dans un conduit corporel (100), et **en ce que** ladite portion distale (3a) dudit premier ballonnet (3) est conformée de manière à entourer une portion proximale de cet embout (12) et, à l'état non gonflé, à s'étendre à proximité de l'extrémité distale (5a) de la gaine (5).

14. Système (1) selon les revendications 1 à 13, **caractérisé en ce qu'**il comporte un élément (60) limitant le gonflage proximal, ce qui permet de s'assurer que le gonflage dudit premier ballonnet (3) à partir de ladite partie distale du ballonnet (3) et progresse dans la direction proximale.

15. Système (1) selon la revendication 14, **caractérisé en ce que** ledit élément de restriction (60) est sous la forme d'un manchon entourant ledit premier ballonnet (3) de manière ajustée, une extrémité distale de ce manchon dépassant de l'extrémité distale (5a) de la gaine (5) et étant en retrait de l'extrémité distale (3b) dudit premier ballonnet (3), et ce manchon étant déchirable longitudinalement sous l'effet du gonflement de ce premier ballonnet (3), de manière à ne pas faire obstacle à ce gonflement.

16. Système (1) selon la revendication 15, **caractérisé en ce que** ledit manchon est en un matériau à faible coefficient de friction, notamment du PTFE (polytétrafluoroéthylène).

17. Système (1) selon la revendication 15 ou la revendication 16, **caractérisé en ce que** ledit manchon comprend une encoche longitudinale (61) aménagée à partir de son extrémité distale, constituant une amorce de déchirure longitudinale.

18. Système (1) selon l'une des revendications 1 à 17, **caractérisé en ce qu'**il inclut un élément (70) d'empêchement ou de restriction de la dilatation radiale de la gaine (5).

19. Système (1) selon la revendication 18, **caractérisé en ce que** ledit élément (70) d'empêchement ou de restriction de la dilatation radiale de la gaine (5) comprend un manchon (70) en un matériau élastiquement déformable, prolongeant ladite extrémité distale (5a) de la gaine (5), et ayant, à l'état de non gonflement dudit premier ballonnet (3), une forme conique convergent radialement, c'est-à-dire s'étendant depuis ladite extrémité distale (5a) de la gaine (5) jusqu'audit premier ballonnet (3), et se dilatant radialement lors du gonflement de ce premier ballonnet (3), pour adopter une forme évasée, s'évasant radialement.

20. Système (1) selon la revendication 18 ou la revendication 19, **caractérisé en ce que** ledit élément (70) d'empêchement ou de restriction de la dilatation radiale de la gaine (5) comprend une bague en un matériau non étirable radialement, fixée au niveau de l'extrémité distale (5a) de la gaine (5).
